# EUROPEAN PATENT SPECIFICATION

(11) **EP 4 025 058 B1**
(45) Date of publication and mention of the grant of the patent: **24.04.2024**
(21) Application number: 20765255.3
(22) Date of filing: 01.09.2020
(51) Int. Cl.: A22B 5/00, G01N 33/12

(54) **AUTOMATIC REMOVAL OF CONTAMINATION ON CARCASSES**
AUTOMATISCHES ENTFERNEN VON VERUNREINIGUNGEN AN SCHLACHTKÖRPER
ENLÈVEMENT AUTOMATIQUE DE CONTAMINATIONS SUR DES CARCASSES

(30) Priority: 02.09.2019 DK PA201901036
(43) Date of publication of application: 13.07.2022
(73) Proprietor: Teknologisk Institut, 2630 Taastrup (DK)
(72) Inventor: HANSEN, Rikke Hjort, 2630 Taastrup (DK); DIRAC, Paul Andreas Holger, 2630 Taastrup (DK); NIELSEN, Glenn Gunner Brink, 2630 Taastrup (DK); MADSEN, Niels Toftelund, 2630 Taastrup (DK); CHRISTENSEN, Lars Bager, 2630 Taastrup (DK); DAM, Jeppe Seidelin, 2630 Taastrup (DK)
(86) International application number: PCT/EP2020/074301
(87) International publication number: WO 2021/043747

(56) References cited:
- WO-A1-99/45138
- WO-A1-2009/138083
- US-B2- 10 222 260

## Description

### TECHNICAL FIELD

This invention relates to a system for identification, removal, and inspection of contaminations on an animal carcass (2), and a corresponding method. The system of the invention comprises the following elements: a detection system, a removal system, an inspection system and a processing means.

The method of the invention involves the use of a detection system to determine the existence and location of contaminations, and to report the existence and location to a processing means, to guide a removal tool to the location of the contamination and remove the contamination.

### BACKGROUND ART

Slaughter carcasses, e.g. bovine and porcine, may be contaminated during the slaughter process. Contaminations can originate from the animal, e.g. faeces, bile or hair, or from the production environment, e.g. grease oil.

Contaminations on the carcass must be removed to ensure consumer safety and health, and to ensure product shelf life and for aesthetic reasons.

The task of ensuring contamination free carcasses requires first detecting the presence of contamination and then removing the contamination, e.g. using steam and suction, or using a knife to trim away contaminated carcass parts. Today operators perform these detection and removal operations. The detecting operator is typically a veterinary inspector who must look for animal sickness, abscesses, broken bones, etc. in addition to detecting contamination. If contamination is detected it is removed by the veterinary inspector or by another operator. This manual approach has several drawbacks:
Inspectors may be distracted or become tired and miss contamination during inspection. E.g. if one part of the carcass needs attention, contamination on another part may be overlooked.

Manual inspection is inherently subjective and will vary over time, between inspectors, and between slaughterhouses and countries.

If the contamination is removed by another operator than the detecting operator, the removing operator must re-locate the contamination, which is inefficient.

Manual inspection and removal are labour intensive and therefor costly. To reduce the risk of missing a contamination, inspectors are typically highly trained and have relatively short duty cycles to avoid fatigue and loss of concentration.

Automation of the process removal of contamination would therefore be advantageous.

WO9945138 describes a method and an apparatus for detecting ingesta or faecal contamination on an animal carcass using fluorescent spectroscopy.

WO2010103261 describes methods for analysing an animal carcass, meat obtained therefrom, or product produced by or obtained from an animal for the presence or absence of faecal matter.

WO199945138 discloses an apparatus for detecting ingesta or faecal contamination on an animal carcass using fluorescent spectroscopy.

WO2009138083 A1 discloses a cleaning device for cleaning the surface of a carcass by contact, the cleaning device comprising a plurality of steam discharge jets and a suction nozzle. A robot having at least two directions of linear movement is disclosed therein and a cleaning device has been mounted on the robot's arm.

However, the system and method described herein, for identification, removal and inspection of contaminations an animal carcass, have never been disclosed.

### SUMMARY OF THE INVENTION

This invention relates to a detection, removal and inspection system for the identification, removal, and inspection of contaminations on an animal carcass.

The method of the invention involves the use of a detection system to determine the existence and location of contaminations, and to report the existence and location to an IT-system (i.e. a processing means), to guide a removal tool to the location of the contamination and remove the contamination, and finally to inspect and verify that any contamination is adequately removed, and to report the result of the inspection to an IT-system so that the carcass may be released or retained for further corrective actions according to the inspection result.

Other objects of the invention will be apparent to the person skilled in the art from reading the following detailed description and accompanying drawings.

### BRIEF DESCRIPTION OF THE DRAWINGS

The present invention is further illustrated by reference to the accompanying drawing, in which:

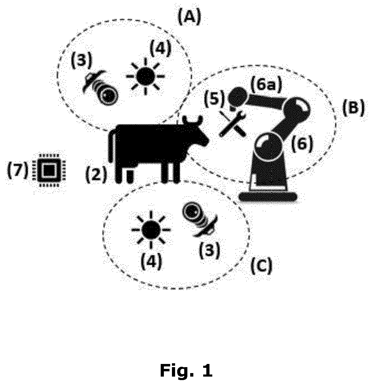

Fig. 1 shows a schematic overview of the system of the invention, wherein:
(A) illustrates the detection system of the invention, in which (3) illustrates one or more camera(s), and (4) illustrates the one or more light sources;
(B) illustrates the removal system of the invention, in which (5) illustrates the removal tool for use according to the invention, and (6) illustrates a robot comprising a robotic arm (6a) for use according to the invention; and
(C) illustrates the inspection system of the invention, wherein (3) illustrates one or more camera(s), (4) illustrates the one or more light sources;

(2) illustrates the object/animal carcass to be analysed; and
(7) illustrates the processing means for use according to the invention.

### DETAILED DISCLOSURE OF THE INVENTION

In its first aspect, the present invention provides a **system** according to claim 1 **for identification, removal and** inspection of **contaminations an animal carcass (2),** comprising the following elements:
i. a **detection system (A),** comprising one or more **cameras** (3), in communication with a processing means (7), and one or more **light sources** (4), optionally in communication with the processing means (7), which detection system is capable of identifying the presence, and of determining and location of any contamination in a three-dimensional (3D) space;
ii. a **removal system (B),** in communication with a processing means (7), and capable of removing any contamination as determined by the detecting system (A) of the invention, and/or capable of trimming away any contaminated areas of the carcass, said removal system including an inspection **system (C),** comprising one or more **cameras** (3), in communication with a processing means (7), and one or more **light sources** (4), optionally in communication with the processing means (7), which inspection system is capable of identifying the presence, and of determining the location of any contamination; and
iii. **a processing means** (7), in communication with the detection system (A) and the removal system (B), including the inspection system (C).

The **removal system** comprises a **removal tool** (5), mounted on a robotic arm (6a), guided by a robot (6) in communication with the processing means (7).

### Detection system

The detection system (A) of the invention may be characterised by comprising one or more RGB cameras (3) with high pixel resolution, aimed to cover the entire carcass (2) with sufficient resolution, and capable of resolving even small contaminations.

The cameras (3) for use according to the invention may be multi-spectral, i.e. they may be able to spectrally resolve several wavelengths to form a multispectral image of the carcass of parts of it. The cameras may be placed in housings that are suitable to protect them from disturbances from slaughterhouse procedures, e.g. from operators in the vicinity, and from cleaning.

To ensure adequate lighting of the carcass (2), so that clear images can be recorded with shutter times short enough to avoid detrimental motion blur in the images, the system of the invention comprises one or more light sources (4). The light for use according to the invention shall have the appropriate wavelengths represented to ensure detection of contamination, and shall also ensure, that different types of contamination can be distinguished by multispectral imaging.

The light sources (4) may be based on light emitting diodes of selected wavelengths. Timing of emission from the different wavelengths may be used to acquire multispectral images, such that one or more wavelengths illuminates the carcass for a very short time, while a camera acquires an image, immediately followed by another image where one or more (other) wavelengths illuminate the carcass.

Images obtained by the cameras (3) are analysed on-line, i.e. right after the image has been taken, using standard image analysis algorithms, designed to detect the manure contamination by marking these with a colour, so location of the contamination is easily identified.

In one embodiment, images are analysed to determine the existence of possible contaminations. Algorithms may e.g. determine the type of contamination, e.g. faecal or oil.

In another embodiment, algorithms are used to categorise contaminations according to which tool is best suited for removal of the contamination in question, e.g. steam vacuum suction or knife trimming.

In a further embodiment, algorithms are used to determine the best path of the removal tool in order to safely remove the contamination.

The algorithms used in data analysis may be a combination of classical image analysis algorithms, artificial neural networks, deep convolutional networks, and reinforcement learning, and machine learning algorithms such as Random Forest, k-Nearest Neighbours, or Support Vector Machines.

The detection system (A) of the invention also comprises means to determine the location of contaminations in the three-dimensional space, e.g. by using stereo vision, structured light, time-of-flight depth measurements, or combinations thereof.

The determination of the 3D location of the contamination may be based on the same cameras that detect the contamination, or it may be based on separate 3D measurement sensors/cameras. In the latter case, the detection cameras and the 3D measurement sensors/cameras must be calibrated, so the location on the 2D detection images can be transform into a 3D location in space.

If the carcass is moving, e.g. on an overhanging rail or on a conveyor belt, the systems may have means to predict the location of the contamination in question at future times after detection, e.g. by measuring the speed of the carcass, or by using information about the speed of the carcass transport means in the slaughterhouse, and/or by means of identity-based tracking systems, such as radio frequency identifiers.

### Removal system

The removal system (B) of the invention comprises one or more removal tools (5) appropriate for removing any contamination, and/or for trimming away any contaminated areas of the carcass. Removal tools (5) for use according to the invention may e.g. be in the form of steam vacuum suction devices, and/or knifes, e.g. knife blades or rotating wizard knives.

The removal tools (5) for use according to the invention shall be directed to the location of the contamination and shall be moved along a path to remove the contamination by means of a mechanical transport device, equipped with one or more removal tools. The device may e.g. be in the form of one or more robotic arms (6a).

The transport device may also be equipped with one or more cameras and/or sensors (3). The cameras/sensors (3) may be used to detect and/or locate the contamination on the carcass, and/or to increase the precision of a predetermined location.

Cameras/sensors (3) may also be used to determine the distance of the transport device and/or removal tool from the carcass. The sensors (3) may in particular be distance sensors and/or force sensors.

### inspection System

The inspection system (C) of the invention comprises one or more cameras (3) aimed to cover the entire carcass (2), with sufficient resolution to be able to resolve even small contaminations. The cameras (3) for use according to the invention may be multi-spectral, i.e. they may be able to spectrally resolve several wavelengths to form a multi-spectral image of the carcass, or of parts of it.

The cameras (3) for use according to the invention may be placed in housings that are suitable to protect them from disturbances from slaughterhouse procedures, e.g. from operators in the vicinity, and/or from cleaning.

In one embodiment, the cameras (3) are placed on one or more transport devices, e.g. on a robotic arm (6a), and may be programmed to inspect the locations of any previously detected and removed contaminations.

The inspection system (C) is included in the removal system (B), so that the removal system (B) performs both removal and inspection of removal.

### Method for identification, removal, and inspection

In another aspect the present invention provides a method for the identification, removal and inspection of contaminations on an animal carcass (2), which method comprises the subsequent steps of:
a. subjecting an object (2) to a detection step for identification of any contaminations using the detection system (A) of the invention;
b. subjecting the object (2) to a removal and/or trimming step using the removal system (B) of the invention; and
c. subjecting the object (2) to an inspection step using the inspection system (C) of the invention.

The object to be analysed (2) according to the method of the invention may be static or may be moving, e.g. along an overhead rail or on a conveyor belt.

In one embodiment, if the carcass is moving, the system comprises means to predict the location of contamination at future times after detection, e.g. by measuring the speed of the moving carcass, by using information about the speed of the carcass transport means used in the slaughterhouse, and/or by means of an identity-based tracking system, e.g. using radio frequency identifiers.

The removal system (B) employed in step b is able to perform both removal and inspection of any contaminations identified on the animal carcass (2).

The object to be analysed (2) according to the method of the invention may be an animal carcass, or meat obtained therefrom, or products produced by or obtained from an animal.

The contaminations sought identified and removed by the method of the invention or by using the system of the invention may originate from the animal, and may e.g. be faeces, bile or hair, or from the production environment, e.g. grease oil, which contaminations should be removed to ensure consumer safety and health, and to ensure product shelf life and for aesthetic reasons.

Any combination of two or more of the embodiments described herein is considered within the scope of the present invention.

### List of reference signs

In the figures, identical structures, elements or parts that appear in more than one figure are generally labelled with the same numeral in all the figures in which they appear.
1. The system of the invention
2. Object to be analysed/animal carcass
3. Camera/sensor
4. Light source
5. Removal tool
6. Robot
6a. Robotic arm
7. Processing means

### EXAMPLES

The invention is further illustrated with reference to the following examples, which are not intended to be in any way limiting to the scope of the invention as claimed.

### Example 1

### Detection of faecal contamination

A picture is taken with a standard industrial RGB camera (Basler camera), with a high pixel resolution, so it was possible to see even very small contaminants, i.e. down to approx. 0.5 mm.

The image was analysed online, i.e. immediately after the picture was taken, and an algorithm detects the fertilizer contamination by plotting these on the picture with a colour, so it is easy to quickly see where on the carcass the manure contamination is located.

The algorithm used for detection is Deep Learning, which uses a Convolutional Neural Network (CNN). It is a pixel-wise fold, i.e. each pixel is classified, but evaluated and within a kernel, i.e. a smaller area, so many factors come into play in relation to whether the pixel in question is manure or not. This know-how is the network, which involves millions of factors to optimize in relation to the task set.

The algorithm is made by training the network by giving it many images, approx. 2000 images, where the manure pollution has been ingested, i.e. any pixel that is manure contaminant is ingested in the image. Thereby a variation is obtained in the model, which will to a large extent correspond to the variance of reality, and thus a data-driven result is obtained, which is considered a good representation of reality.

The network is based on known algorithms and networks available with e.g. Tensor Flow (from Google) and PyTorch (from Facebook).

For photographing the pigs, a multispectral camera was used, i.e. a standard RGB camera and a near-infrared camera, whereby an image with a wavelength outside the human visible area is obtained. This picture has four values in each pixel in the image, i.e. four wavelength ranges: red, green, blue and near-infrared.

A Random Forests method was used, which is an ensemble method within Machine Learning. Each pixel is assessed in relation to a number of factors, simultaneous with including surrounding pixel information.

The pig's skin has the cleaned by burning, so manure contamination often occurs inside, when the fat end is taken out, which is done by a machine. Occurrence of the manure contamination is approx. 2% of all pig half-carcasses. In comparison, the incidence of manure on cattle is approx. 14% of all bovine carcasses.

## Claims

1. **A system for the identification, removal, and control of contaminations on an animal carcass (2),** which system comprises the following elements:
i. a processing means (7); ii. **a detection system (A),** comprising one or more **cameras** (3), in communication with the processing means (7), and one or more **light sources** (4), optionally in communication with the processing means (7), which detection system is capable of identifying and determining the presence and location of any contamination in a three-dimensional (3D) space;
iii. **a removal system (B)** comprising a **removal tool** (5), mounted on a robotic arm (6a), guided by a robot (6), in communication with the processing means (7), and capable of removing or trimming away any contamination as determined by the detecting system (A), said removal system (B) including **an inspection system (C),** comprising one or more **cameras** (3), in communication with the processing means (7), and one or more **light sources** (4), optionally in communication with the processing means (7), which inspection system is capable of identifying the presence, and of determining the location of any contamination on the animal carcass;
wherein the removal system (B) is arranged to perform both removal of any contaminations, and inspection of this removal.

2. The system of claim 1, wherein the removal tool (5) is a steam vacuum suction device, and/or a knife, e.g. knife blades or rotating wizard knives.

3. The system of either one of claims 1-2, which system comprises means to predict the location of contamination on an incoming animal carcass at future times after detection.

4. The system of any one of claims 1-3, for the identification, removal and control of contaminations originating from the animal carcass, and which may e.g. be faeces, bile or hair, or contaminations originating from the production environment, e.g. grease oil.

5. **A method of use of the system of** any one of claims 1 to 4, **for the identification, removal, and control of contaminations on an animal carcass (2),** which method comprises the subsequent steps of:
a. subjecting an animal carcass (2) to a detection step for identification of any contaminations using the detection system (A) comprising one or more cameras (3), in communication with the processing means (7), and one or more light sources (4), optionally in communication with the processing means (7), which detection system is capable of identifying and determining the presence and location of any contamination in a three-dimensional (3D) space; and
b. subjecting the object (2) to a removal and/or trimming step using the removal system (B) in communication with the processing means (7), and capable of removing or trimming away any contamination as determined by the detecting system (A); which method further requires that the removal system (B) employed in step b performs both removal and a subsequent inspection of contaminations on the animal carcass,

6. The method of claim 5, wherein the animal carcass to be analysed (2) may be static or may be moving.

7. The method of claim 6, wherein, if the animal carcass is moving, the system comprises means to predict the location of contamination at future times after detection, e.g. by measuring the speed of the moving carcass, by using information about the speed of the carcass transport means, and/or by means of an identity-based tracking system.

## Patentansprüche

1. **Ein System zur Identifizierung, Entfernung und Kontrolle von Verunreinigungen an einem Tierkadaver (2),** wobei das System die folgenden Elemente umfasst:
i. **ein Verarbeitungsmittel** (7);
ii. **ein Erkennungssystem (A),** umfassend eine oder mehrere Kameras (3), die mit den Verarbeitungsmitteln (7) kommunizieren, und eine oder mehrere Lichtquellen (4), optional in Kommunikation mit den Verarbeitungsmitteln (7), welches Erkennungssystem ist in der Lage, das Vorhandensein und den Ort einer Kontamination in einem dreidimensionalen (3D) Raum zu identifizieren und zu bestimmen;
iii. **ein Entfernungssystem (B),** das ein **Entfernungswerkzeug** (5) umfasst, das an einem Roboterarm (6a) montiert ist, von einem Roboter (6) geführt wird, mit dem Verarbeitungsmittel (7) kommuniziert und in der Lage ist, jegliche Kontamination zu entfernen oder wegzuschneiden wie durch das Erkennungssystem (A) bestimmt,
wobei das Entfernungssystem (B) **ein Inspektionssystem (C)** umfasst, das eine oder mehrere Kameras (3) in Verbindung mit dem Verarbeitungsmittel (7) und eine oder mehrere **Lichtquellen** (4) umfasst, optional in Verbindung mit dem Verarbeitungsmittel (7), welches Inspektionssystem in der Lage ist, das Vorhandensein jeglicher Kontamination am Tierkadaver zu erkennen und den Ort zu bestimmen;
wobei das Entfernungssystem (B) so angeordnet ist, dass es sowohl die Entfernung etwaiger Verunreinigungen als auch die Inspektion dieser Entfernung durchführt.

2. System nach Anspruch 1, wobei das Entfernungswerkzeug (5) ein Dampf-Vakuum-Sauggerät und/oder ein Messer ist, z.B. eine Messerklinge oder rotierende Zaubermesser.

3. Das System nach einem der Ansprüche 1 bis 2, wobei das System Mittel umfasst, um den Ort der Kontamination auf einem ankommenden Tierkadaver zu zukünftigen Zeitpunkten nach der Erkennung vorherzusagen.

4. Das System nach einem der Ansprüche 1 bis 3 zur Identifizierung, Entfernung und Kontrolle von Verunreinigungen, die aus dem Tierkadaver stammen und z. B. Fäkalien, Galle oder Haare, oder Verunreinigungen aus der Produktionsumgebung, z.B. Fettöl.

5. **Verfahren zur Verwendung des Systems nach einem der Ansprüche 1 bis 4 zur Identifizierung, Entfernung und Kontrolle von Verunreinigungen auf einem Tierkadaver (2),** wobei das Verfahren die folgenden Schritte umfasst:
a. unterziehen eines Tierkadavers (2) einem Detektionsschritt zur Identifizierung etwaiger Kontaminationen unter Verwendung des Detektionssystems (A), das eine oder mehrere Kameras (3) in Kommunikation mit der Verarbeitungseinrichtung (7) und eine oder mehrere Lichtquellen (4) umfasst, optional in Kommunikation mit den Verarbeitungsmitteln (7), wobei das Erkennungssystem in der Lage ist, das Vorhandensein und den Ort einer Kontamination in einem dreidimensionalen (3D) Raum zu identifizieren und zu bestimmen; und
b. unterziehen des Objekts (2) einem Entfernungs- und/oder Beschneidungsschritt unter Verwendung des Entfernungssystems (B), das mit der Verarbeitungseinrichtung (7) in Verbindung steht und in der Lage ist, jedwede durch das Erkennungssystem (A) ermittelte Kontamination zu entfernen oder zu beschneiden;
dieses Verfahren erfordert außerdem, dass das in Schritt b eingesetzte Entfernungssystem (B) sowohl die Entfernung als auch eine anschließende Inspektion der Verunreinigungen am Tierkadaver durchführt.

6. Verfahren nach Anspruch 5, wobei der zu analysierende Tierkadaver (2) statisch sein oder sich bewegen kann.

7. Verfahren nach Anspruch 6, wobei das System, wenn sich der Tierkadaver bewegt, Mittel umfasst, um den Ort der Kontamination zu zukünftigen Zeitpunkten nach der Erkennung vorherzusagen, z. B. durch Messung der Geschwindigkeit des sich bewegenden Kadavers, durch Nutzung von Informationen über die Geschwindigkeit des Kadavertransportmittels und/oder mittels eines identitätsbasierten Trackingsystems.

## Revendications

1. **Système d'identification, d'élimination et de contrôle des contaminations sur une carcasse d'animal (2),** lequel système comprend les éléments suivants :
i. **un moyen de traitement** (7) ;
ii. **un système de détection (A),** comprenant une ou plusieurs caméras (3), en communication avec les moyens de traitement (7), et une ou plusieurs **sources lumineuses** (4), éventuellement en communication avec les moyens de traitement (7), lequel système de détection est capable d'identifier et de déterminer la présence et l'emplacement de toute contamination dans un espace tridimensionnel (3D);
iii. **un système de retrait (B)** comprenant un **outil de retrait** (5), monté sur un bras robotique (6a), guidé par un robot (6), en communication avec les moyens de traitement (7), et capable de retirer ou d'éliminer toute contamination tel que déterminé par le système de détection (A),
ledit système d'enlèvement (B) comprenant **un système d'inspection** (C), comprenant une ou plusieurs **caméras** (3), en communication avec les moyens de traitement (7), et une ou plusieurs **sources de lumière** (4), éventuellement en communication avec les moyens de traitement (7), quel système d'inspection est capable d'identifier la présence et de déterminer la localisation de toute contamination sur la carcasse de l'animal;
le système d'élimination (B) étant agencé pour effectuer à la fois l'élimination de toute contamination et l'inspection de cette élimination.

2. Système selon la revendication 1, dans lequel l'outil de retrait (5) est un dispositif d'aspiration de vapeur et/ou un couteau, par ex. lames de couteau ou couteaux de sorcier rotatifs.

3. Système selon l'une ou l'autre des revendications 1 et 2, lequel système comprend des moyens pour prédire l'emplacement de la contamination sur une carcasse d'animal entrante à des moments ultérieurs après la détection.

4. Système selon l'une quelconque des revendications 1 à 3, pour l'identification, l'élimination et le contrôle des contaminations provenant de la carcasse d'un animal, et qui peut par ex. être des matières fécales, de la bile ou des cheveux, ou des contaminations provenant de l'environnement de production, par ex. huile de graisse.

5. **Procédé d'utilisation du système selon l'une quelconque des revendications 1 à 4, pour l'identification, l'élimination et le contrôle des contaminations sur une carcasse d'animal (2),** lequel procédé comprend les étapes suivantes:
a. soumettre une carcasse d'animal (2) à une étape de détection d'identification d'éventuelles contaminations à l'aide du système de détection (A) comprenant une ou plusieurs caméras (3), en communication avec les moyens de traitement (7), et une ou plusieurs sources lumineuses (4), éventuellement en communication avec les moyens de traitement (7), lequel système de détection est capable d'identifier et de déterminer la présence et la localisation de toute contamination dans un espace tridimensionnel (3D); et
b. soumettre l'objet (2) à une étape de retrait et/ou de rognage à l'aide du système de retrait (B) en communication avec les moyens de traitement (7), et capable d'éliminer ou de rogner toute contamination telle que déterminée par le système de détection (A);
lequel procédé nécessite en outre que le système d'élimination (B) utilisé à l'étape b effectue à la fois l'élimination et une inspection ultérieure des contaminations sur la carcasse de l'animal.

6. Procédé selon la revendication 5, dans lequel la carcasse d'animal à analyser (2) peut être statique ou mobile.

7. Procédé selon la revendication 6, dans lequel, si la carcasse d'animal est en mouvement, le système comprend des moyens pour prédire l'emplacement de la contamination à des moments ultérieurs après la détection, par ex. en mesurant la vitesse de la carcasse en mouvement, en utilisant des informations sur la vitesse du moyen de transport de la carcasse, et/ou au moyen d'un système de suivi basé sur l'identité.
